# EUROPEAN PATENT SPECIFICATION

(11) **EP 1 640 722 B1**
(45) Date of publication and mention of the grant of the patent: **29.10.2008**
(21) Application number: 05021027.7
(22) Date of filing: 27.09.2005
(51) Int. Cl.: G01N 33/86, C12Q 1/56

(54) **Method of inactivating a blood coagulation factor**
Verfahren zur Inaktivierung eines Blutgerinnungsfaktor
Méthode pour l'inactivation d'un facteur de coagulation sanguine

(30) Priority: 27.09.2004 JP 2004278851
(43) Date of publication of application: 29.03.2006
(73) Proprietor: SYSMEX CORPORATION, Kobe-shi, Hyogo 651-0073 (JP)
(72) Inventor: Okuda, Masahiro, Kobe-shi, Hyogo 651-2244 (JP); Yamamoto, Yoshihito, Akashi-shi, Hyogo 673-0011 (JP); Yoshioka, Akira, Nara-shi, Nara 630-8301 (JP); Shima, Midori, Nara-shi, Nara 630-8307 (JP); Takeyama, Masahiro, Kashihara-shi, Nara 634-0811 (JP)
(74) Representative: HOFFMANN EITLE

(56) References cited:
- EP-A- 0 391 974
- EP-A- 0 711 838
- US-B1- 6 211 344
- DUGA STEFANO ET AL: "Coagulation factor V" INTERNATIONAL JOURNAL OF BIOCHEMISTRY & CELL BIOLOGY, vol. 36, no. 8, August 2004 (2004-08), pages 1393-1399, XP002360408 ISSN: 1357-2725
- BHOPALE G M ET AL: "Blood coagulation factor VIII: An overview." JOURNAL OF BIOSCIENCES. DEC 2003, vol. 28, no. 6, December 2003 (2003-12), pages 783-789, XP002360410 ISSN: 0250-5991
- WALKER F J: "Identification of a new protein involved in the regulation of the anticoagulant activity of activated protein C: Protein S-binding protein" JOURNAL OF BIOLOGICAL CHEMISTRY 1986 UNITED STATES, vol. 261, no. 23, 1986, pages 10941-10944, XP002360409
- LOLLAR P.; HILL-EUBANKS D.C.; PARKER C.G.: 'Association of the factor VIII light chain with von Willebrand factor' JOURNAL OF BIOLOGICAL CHEMISTRY vol. 263, no. 21, 25 July 1988, BIRMINGHAM, US, pages 10451 - 10455, XP001148487
- PROESCHER F.: 'Anti-coagulant properties of ethylene bis-iminodiacetic acid' PROCEEDINGS OF THE SOCIETY FOR EXP. BIOLOGY AND MEDICINE vol. 76, no. 4, 01 April 1951, SOCIETY FOR EXPERIMENTAL BIOLOGY AND MEDICINE (NEW YORK, N.Y.), pages 619 - 620, XP008057276

## Description

### BACKGROUND OF THE INVENTION

### 1. Field of the Invention

The present invention relates to a method of inactivating a blood coagulation factor. The present invention also relates to a blood coagulation factor-inactivated sample containing a blood coagulation factor in an inactivated form. Further, the present invention relates to a method of measuring the activity of a blood coagulation factor by using blood coagulation factor-inactivated plasma containing a blood coagulation factor in an inactivated form. In addition, the present invention relates to a blood sample treating agent obtained by using the blood coagulation factor-inactivated plasma.

### 2. Description of the Related Art

Blood coagulation examination is carried out to examine congenital or postnatal blood coagulation abnormality. Hemophilia famous as congenital coagulation abnormality is a disease occurring due to deficiency in the activity of factor VIII or IX among blood coagulation factors.

Examination of blood coagulation abnormality includes measurement of activated partial thromboplastin time (APTT), measurement of prothrombin time (PT), quantification of fibrinogen, etc. In order to examine the cause of the abnormalities in blood coagulation, it is necessary to measure each blood coagulation factor in a blood sample. The method of measuring blood coagulation factor includes measurement of the activity of a blood coagulation factor based on APTT and PT. In the measurement, a blood sample to be examined, plasma deficient in a certain blood coagulation factor and a reagent for measuring a coagulation time are mixed, and the coagulation time is measured, and then the activity of the certain coagulation factor is calculated based on the coagulation time. Accordingly, plasma deficient in a coagulation factor as a subject of examination (coagulation factor-deficient plasma) is necessary for examination of blood coagulation.

Plasma from mammals such as humans with congenital deficiency, or plasma from which a certain coagulation factor was removed from normal plasma by a method of immune adsorption with an antibody binding to the predetermined coagulation factor (artificial deficiency plasma), is used as the coagulation factor-deficient plasma used in measurement of APTT and PT.

The artificial deficiency blood is increasingly utilized from the viewpoint of easy acquisition. However, artificial removal of a blood coagulation factor by the method of immune adsorption has problems such as difficult preparation of an antibody used, removal of blood coagulation factor V, factor VIII or von Willebrandt factor (VWF), and higher production costs due to the necessity for a longer time of adsorption removal with using a large amount of antibody.

Besides the method of immune adsorption, JP-A 2002-90361 proposes a method of removing a blood coagulation factor through adsorption by mixing human plasma with a synthetic polymer. However, the method described herein is a method of removing a blood coagulation factor through adsorption, thus suffering from the problem that factor V, factor VIII, or VWF is removed.

### SUMMARY OF THE INVENTION

The present invention was made in view of the circumstances described above, and an object of the present invention is to provide a method of inactivating a blood coagulation factor in a sample, that is, a novel method completely different from the conventional method of removing a blood coagulation factor by adsorption. Another object of the invention is to provide a blood coagulation factor-inactivated sample containing a blood coagulation factor in an inactivated form, that is, a novel sample completely different from a sample deficient in a blood coagulation factor such as the conventional coagulation factor-deficient plasma. A further object of the invention is to provide a method of measuring the activity of a blood coagulation factor by using blood coagulation factor-inactivated plasma containing a blood coagulation factor in an inactivated form. A still further object of the invention is to provide a blood sample treating agent obtained by using blood coagulation factor-inactivated plasma.

A first aspect of the present invention relates to a method of inactivating a blood coagulation factor, comprising a step of contacting a sample containing at least one of factors V and VIII with a compound having an iminodiacetate group -N(CH₂COOR)₂ wherein R represents a hydrogen atom or a metallic ion, whereby at least one of factors V and VIII in the sample is changed into an inactivated form.

A second aspect of the present invention relates to a blood coagulation factor-inactivated sample, comprising at least one of factors V and VIII in an inactivated form.

A third aspect of the present invention relates to a method of measuring an activity of a blood coagulation factor contained in a blood sample, comprising the steps of: (a) preparing mixture by mixing the blood sample, a measurement reagent for measuring a coagulation time and a blood sample treating agent comprising blood coagulation factor-inactivated plasma containing factors V and VIII in an inactivated form; (b) measuring a coagulation time in the mixture; and (c) calculating an activity of factor V or VIII on the basis of the coagulation time.

A forth aspect of the present invention relates to a blood sample treating agent, comprising: (a) blood coagulation factor-inactivated plasma containing factors V and VIII in an inactivated form; and (b) factor V changeable into an activated form or factor VIII changeable into an activated form.

### BRIEF DESCRIPTION OF THE DRAWINGS

Fig. 1 is a graph showing measurement results of the activity of factor VIII in plasma obtained by contact treatment with a different kind of cation exchange resin.
Fig. 2 is a graph showing measurement results of the activity of factor V in plasma obtained by contact treatment with a different kind of cation exchange resin.
Fig. 3 is a graph showing measurement results of the activity of factor VIII in plasma obtained by contact treatment with cation exchange resin having an iminodiacetate group.
Fig. 4 is a graph showing measurement results of the activity of factor V in plasma obtained by contact treatment with cation exchange resin having an iminodiacetate group.
Fig. 5 is a graph showing measurement results of the activity of factor VIII in plasma obtained by contact treatment of factor V-deficient plasma with cation exchange resin having an iminodiacetate group.
Fig. 6 is a graph showing measurement results of the activity of factor V in plasma obtained by contact treatment of factor VIII-deficient plasma with cation exchange resin having an iminodiacetate group.
Fig. 7A is a photograph showing results of electrophoresis conducted under non-reducing conditions.
Fig. 7B is a photograph showing results of electrophoresis conducted under reducing conditions.
Fig. 8 is a graph showing a measurement result of the activities of factors VIII and V in normal canine plasma.
Fig. 9 is a graph showing a measurement result of the activities of factors V and VIII in plasma obtained by contact treatment of normal canine plasma with cation exchange resin having an iminodiacetate group.
Fig. 10 is a graph showing measurement results of the activity of factor VIII in plasma obtained by contact treatment with Sepharose having an iminodiacetate group.
Fig. 11 is a graph showing measurement results of the activity of factor V in plasma obtained by contact treatment with Sepharose having an iminodiacetate group.

### DESCRIPTION OF THE PREFERRED EMBODIMENTS

The method of inactivating a blood coagulation factor according to the present invention comprises a step of contacting a sample with a compound having an iminodiacetate group, whereby at least one of factors V and VIII in the sample is changed into an inactivated form.

Herein, the sample is not particularly limited insofar as it can contain at least one of factors V and VIII, and examples of the sample include plasma, serum and whole blood from mammals such as humans. Other examples include solutions containing a coagulation factor product of factor V, factor VIII, factor VIII/VWF complex, etc. The coagulation factor product is obtained by extraction or purification from blood or prepared by genetic recombination technique.

The iminodiacetate group is -N(CH₂COOR)₂ wherein R represents a hydrogen atom or a metallic ion. The group R is preferably a metallic ion, more preferably a monovalent metallic ion. The monovalent metallic ion includes, for example, sodium ion and potassium ion.

Insofar as the compound has the iminodiacetate group as a functional group, the compound is not particularly limited and may be an organic or inorganic compound. The compound also includes compounds to which an iminodiacetate group is bound directly or indirectly via a spacer, a coupling agent or the like by various methods of introducing the functional group. Essentially, the compound may be any compound having an iminodiacetate group, which when used in contact treatment, can inactivate at least one of factors V and VIII. Such compound includes, for example, carriers. Specific examples include carriers composed of organic compounds, such as cation exchange resin and granular agarose gel, and carriers composed of inorganic compounds, such as magnetic beads, silica particles, and a monolithic silica column.

The cation exchange resin having an iminodiacetate group is a 3-dimensional reticulated resin which is insoluble in water or a solvent, contains an iminodiacetate group as a functional group capable of cation exchange, and can selectively adsorb an alkali metal, an alkaline earth metal, a heavy metal etc.

The cation exchange resin having an iminodiacetate group may be porous spherical particles (beads type) or a film having microscopic voids (ion exchange membrane). The polymer matrix on which the cation exchange resin is based is not particularly limited, and for example, a styrene/divinyl benzene copolymer is used. The ion exchange membrane may be a heterogeneous type membrane formed by dispersing fine powder of cation exchange resin in a colloidal state with a membrane-forming binder (thermoplastic or thermosetting resin such as polyethylene, polystyrene, phenol resin etc.) or a semi-homogeneous type membrane formed by mixing a polymer (polyethylene, polypropylene, polyvinyl chloride, fluorine resin etc.) for maintaining a membrane shape, with a polymer having an cation-exchange group (polystyrene derivative, polyacrylic acid, polyethylene imine, polyvinyl pyridine derivative etc.) or crosslinking the two, or a homogeneous type membrane formed by introducing an cation-exchange group into a styrene/butadiene copolymer membrane or by forming a thermoplastic polymer containing an cation exchange group into a membrane.

The granular agarose gel having an iminodiacetate group is agarose gel formed in a granular form containing iminodiacetic acid as a functional group. Specific examples include Sepharose manufactured by Amersham Biosciences and Bio Gel manufactured by Bio Rad.

The method of contact treatment for contacting a sample with a compound having an iminodiacetate group is not particularly limited. When the compound having an iminodiacetic acid is for example a carrier having an iminodiacetate group, there is a method wherein the carrier and a sample are placed in a container and mixed under stirring for a predetermined time, or a method wherein a sample is passed through a column charged with the carrier.

The amount of the compound having an iminodiacetate group, used in contact treatment, is predetermined suitably depending on the type of the compound. For example, when the compound having an iminodiacetate group is a carrier having an iminodiacetate group, the contact treatment is conducted preferably such that the amount of the carrier is 5% (w/v) or more relative to the sample. When the amount is 5% (w/v) or less, a change of the blood coagulation factor into an inactivated form may be insufficient and the treatment may require a longer time.

The time of contact treatment is predetermined suitably depending on the type and amount of the iminodiacetate group-containing compound used. For example, when the compound having an iminodiacetic acid is a carrier having an iminodiacetate group, the contact time is preferably 2 to 6 hours when the ratio of the carrier to a sample is about 5% (w/v), and the contact time is preferably about 1 to 4 hours when the ratio of the carrier to a sample is about 10% (w/v).

By the contact treatment of contacting a sample with the compound having an iminodiacetate group, at least one of factors V and VIII in the sample can be changed easily and efficiently into an inactivated form. The blood coagulation factor in an inactivated form is a blood coagulation factor which cannot be changed into an activated form.

The factor V is a glycoprotein having a molecular weight of 330,000, and acts as a coenzyme which together with Xa, prothrombin, phospholipid and Ca2+, forms a prothrombinase complex to enhance the activation of prothrombin by Xa. The factor V is cleaved at Arg (position 709), Arg (position 1018) and Arg (position 1545) at the C-terminal side by thrombin, to become activated factor V. However, the factor V inactivated by the contact treatment described above cannot be changed into an activated form. Although what chemical change in the factor V is caused by the contact treatment is not revealed, it is estimated that a factor V moiety to be changed into an activated form is changed chemically or stereo-structurally, or a part of the moiety to be changed into an activated form is defected, or the moiety to be changed into an activated form undergoes influence of a chemical change in another moiety or defection of a part of the moiety.

The factor VIII is a glycoprotein having a molecular weight of 330,000, and acts as a coenzyme which together with IXa, X, phospholipid and Ca2+, forms a tennase complex to enhance the activation of factor X by IXa. The factor VIII is cleaved at Arg (position 372), Arg (position 740) and Arg (position 1689) at the C-terminal side by thrombin, to become activated factor VIII. However, the factor VIII inactivated by the contact treatment described above cannot be changed into an activated form. Although what chemical change in the factor VIII is caused by the contact treatment is not revealed, it is estimated that a factor VIII moiety to be changed into an activated form is changed chemically or stereo-structurally, or a part of the moiety to be changed into an activated form is removed, or the moiety to be changed into an activated form undergoes influence of a chemical change in another moiety or defection of a part of the moiety. It is however estimated that the contact treatment does not influence the ability of factor VIII to bind to VWF. This is because a sample obtained by the contact treatment contains VWF, and the VWF can exhibit a ristocetin cofactor activity, similar to the case where the factor VIII is normal.

In this manner, in the method of inactivating the blood coagulation factor, the factor V or VIII in a sample is changed into an inactivated form by the contact treatment, and thus the factor V or VIII in the sample does not show its activity. That is, the factor V or VIII changed into an inactivated form remains in the sample according to the method of the invention, unlike the conventional method of immune adsorption wherein the factor V or VIII is removed by adsorption. The factor V or VIII remains in the sample in such a form that an antibody against the factor V or VIII reacts therewith.

The factors V and VIII are independently influenced to each other by the contact treatment. Accordingly, the method involving the contact treatment can be utilized to obtain a sample wherein at least one of factors V and VIII has been changed into an inactivated form (referred to hereinafter as blood coagulation factor-inactivated sample), without influencing other factors.

Particularly when plasma is used as a sample, plasma wherein at least one of factors V and VIII has been changed into an inactivated form (referred to hereinafter as blood coagulation factor-inactivated plasma) can be obtained. For example, when normal plasma (plasma containing the factors V and VIII) is subjected to the contact treatment, blood coagulation factor-inactivated plasma wherein the factors V and VIII have been inactivated can be obtained. The factor V or VIII in the blood coagulation factor-inactivated plasma has been changed into an inactivated form, but remains in such a form that an antibody against the factor V or VIII can react therewith. Further, VWF remains in the blood coagulation factor-inactivated plasma, and the factor VIII in the blood coagulation factor-inactivated plasma can bind to VWF to form a complex.

VWF in the blood coagulation factor-inactivated plasma may or may not have a ristocetin cofactor (Rco) activity, and VWF may be chemically or stereo-structurally changed or partially defected. However, when the blood coagulation factor-inactivated plasma is utilized in diagnostic measurement of blood coagulation factor deficiency/abnormality, VWF is preferably the one having an Rco activity. The contact treatment can change at least one of factors V and VIII into an inactivated form, without influencing other factors. Therefore, the contact treatment can be used to obtain blood coagulation factor-inactivated plasma having an Rco activity.

Here, VWF is involved in platelet adhesion at an initial stage of hemostasis as a binding adherent factor intervening between platelet membrane protein GPIb and intravascular subcutaneous tissues. In circulating blood, VWF forms a complex via a non-covalent bond with the factor VIII, thus contributing to stabilization of the factor VIII.

The stability of factor VIII is lost and its blood level is decreased by qualitative and quantitative abnormality in VWF, so that in the examination, prolongation of bleeding time, a reduction or abnormality in the amount of VWF, a reduction in Rco activity, and a reduction in the agglutination ability of platelets with ristocetin are observed. The von Willebrandt disease showing these symptoms is different from hemophilia A caused by a reduction in the activity of factor VIII. Accordingly, when the blood coagulation factor-inactivated plasma is utilized in diagnostic measurement of blood coagulation factor deficiency/abnormality, specifically in measurement of the activity of a blood coagulation factor, it is preferable for accurate judgment of hemophilia caused by deficiency in the activity of factor VIII that in the blood coagulation factor-inactivated plasma, VWF occurs without loosing its activity and simultaneously the factor VIII only is inactivated.

In coagulation factor-deficient plasma produced by the conventional method of removal by adsorption, however, the factor VIII contained in plasma used as a starting material (starting plasma) is removed by adsorption, and thus factor VIII-bound VWF hardly remains. Accordingly, when the produced coagulation factor-deficient plasma is used in measurement of the activity of blood coagulation factor, VWF should be separately added to the plasma prior to measurement. However, it is not necessary to add VWF separately to the blood coagulation factor-inactivated plasma described above.

As described above, the blood coagulation factor-inactivated plasma can be utilized in measurement of the activity of blood coagulation factor. For Example, the blood coagulation factor-inactivated plasma wherein the factors V and VIII have been inactivated can be utilized in measurement of the activity of factor V or VIII in a blood sample.

The measurement comprises the steps of (a) preparing mixture by mixing the blood sample, a measurement reagent for measuring a coagulation time and a blood sample treating agent comprising blood coagulation factor-inactivated plasma, (b) measuring a coagulation time in the mixture and (c) calculating an activity of factor V or VIII on the basis of the coagulation, and the blood sample treating agent comprises blood coagulation factor-inactivated plasma containing factors V and VIII in an inactivated form.

The activity of factor V or VIII in the blood sample can be calculated by comparing the coagulation time of the blood sample to a normal blood sample or a standard blood sample. The normal blood sample includes, for example, a blood collected from a healthy person and a plasma prepared from the blood. The standard blood sample includes, for example, a commercially available standard blood and a standard plasma. The presence or absence of factor V or VIII in the blood sample can be known by the presence or absence of the activity of factor V or VIII. Also, the amount of factor V or VIII present in the blood sample can be known by the degree of activity of factor V or VIII. In addition, the presence or absence of an abnormality in factor V or VIII in the blood sample can be known by the presence or absence of factor V or VIII or the amount thereof.

As the measurement reagent, a PT measurement reagent for measuring PT or an APTT measurement reagent for measuring APTT can be used.

As the blood sample, whole blood or plasma can be used. Which of whole blood and plasma should be selected is determined suitably depending on the reagent and apparatus used in the measurement. When the coagulation time is measured on the basis of optical information such as absorbance, plasma is desirably used as the blood sample.

PT is a time having elapsed from addition of tissue thromboplastin and calcium to a blood sample to coagulation of the blood sample. This coagulation time is prolonged by a deficiency or abnormality in factors I (fibrinogen), II (prothrombin), V, VII or X

The PT measurement reagent contains tissue thromboplastin and calcium as major ingredients. The tissue thromboplastin used can be obtained by extraction or purification from the brain or placenta of a rabbit, cattle or human, or can be prepared by genetic recombination techniques. In addition to the tissue thromboplastin and calcium, a buffer solution, a preservative, a stabilizer etc. may be added.

APTT is a time having elapsed from addition of a phospholipid, an activator and calcium to a blood sample to coagulation of the blood sample. This coagulation time is prolonged by a deficiency or abnormality in factors I, II, V, VIII, IX, X, XI, XII or VWF.

The ATPP measurement reagent contains a phospholipid, an activator and calcium as major ingredients. The phospholipid used may be a synthetic phospholipid, may be obtained by extracting it with an organic solvent from a rabbit or bovine brain or a human placenta, or may be derived from soybeans. The activator in the APTT reagent is the one activating the factors XI and XII, and examples of the activator include kaolin, Celite, silica, ellagic acid, etc. In addition to the major ingredients described above, a buffering agent, a preservative and a stabilizer may be added.

Specific modes of the measurement method include the following modes (I) to (IV). Hereinafter, specific modes wherein plasma is used as the blood sample are shown.
(I) Blood coagulation factor-inactivated plasma containing factors V and VIII in an inactivated form (factors V and VIII-inactivated plasma) is used as the blood sample treating agent and a PT measurement reagent is used as the measurement reagent, to measure the activity of factor V.
   Specifically, factors V and VIII-inactivated plasma is mixed with normal plasma, and the PT measurement reagent is added to the resulting mixture, to measure the coagulation time. In this mixture, serially diluted normal plasma is used to prepare a calibration curve showing the relationship between the activity of factor V and the coagulation time. Separately, the PT measurement reagent is added to a mixture (plasma mixture) comprising plasma as a blood sample mixed with factors V and VIII-inactivated plasma, to measure the coagulation time. On the basis of the previously prepared calibration curve and the coagulation time of the plasma mixture, the activity of factor V contained in the plasma can be calculated.
(II) Factors V and VIII-inactivated plasma to which factor VIII changeable into an activated form was added is used as the blood sample treating agent and a PT measurement reagent is used as the measurement reagent, to measure the activity of factor V
   The blood sample treating agent comprising factor VIII changeable into an activated form added to factors V and VIII-inactivated plasma is plasma wherein factor V is in an inactivated form and factor VIII can be changed into an activated form. The PT measurement reagent is added to a mixture (plasma mixture) comprising plasma as a blood sample mixed with this blood sample treating agent, to measure the coagulation time. On the basis of a calibration curve prepared using serially diluted normal plasma and the coagulation time of the plasma mixture, the activity of factor V contained in the plasma can be calculated.
(III) Factors V and VIII-inactivated plasma to which factor V changeable into an activated form was added, is used as the blood sample treating agent and an APPT measurement reagent is used as the measurement reagent, to measure the activity of factor VIII.
   That is, the blood sample treating agent comprising factor V changeable into an activated form added to factors V and VIII-inactivated plasma is plasma wherein factor VIII is in an inactivated form and factor V can be changed into an activated form. The APPT measurement reagent is added to a mixture (plasma mixture) comprising plasma as a blood sample mixed with this blood sample treating agent, to measure the coagulation time. On the basis of a calibration curve prepared using serially diluted normal plasma and the coagulation time of the plasma mixture, the activity of factor VIII contained in the plasma can be calculated.
(IV) Factors V and VIII-inactivated plasma to which factor VIII changeable into an activated form was added is used as the blood sample treating agent and an APPT measurement reagent is used as the measurement reagent, to measure the activity of factor V.

That is, the blood sample treating agent comprising factor VIII changeable into an activated form added to factors V and VIII-inactivated plasma is plasma wherein factor V is in an inactivated form and factor VIII can be changed into an activated form. The APPT measurement reagent is added to a mixture (plasma mixture) comprising plasma as a blood sample mixed with this blood sample treating agent, to measure the coagulation time. On the basis of a calibration curve prepared using serially diluted normal plasma and the coagulation time of the plasma mixture, the activity of factor V contained in the plasma can be calculated.
Measurement methods in such modes as described above can be used in place of the conventional method of measuring a coagulation factor by using coagulation factor-deficient plasma.

For measurement of the activity of blood coagulation factor, the degree of inactivation of the factor V or VIII in the blood sample treating agent or the blood coagulation factor-inactivated plasma is preferably 80 % or more, more preferably 95 % or more, most preferably 99% or more, assuming that the factor in an activated form contained in a sample before subjection to the contact treatment to be 100%.

Further, the blood sample treating agent or the blood coagulation factor-inactivated plasma can be combined with a conventional PT measurement reagent to constitute a PT measurement reagent kit. Also, the blood sample treating agent or the blood coagulation factor-inactivated plasma can be combined with a conventional APPT measurement reagent to constitute an APTT measurement reagent kit.

Hereinafter, the measurement methods in the Examples will be described.

### (1) Measurement of the activity of factor V

5 *µ*l measurement sample was mixed with 45 *µ*l Owren Veronal buffer solution, and 50 *µ*l of factor V-deficient plasma (Sysmex Corporation) was added thereto and heated at 37°C for 1 minute. Then, 100 µl Thrombocheck PT (Sysmex Corporation) was added to the mixture after heating, and the coagulation time was measured by a blood coagulation analyzer Coaglex 800 (Shimadzu Corporation).

### (2) Measurement of the activity of factor VIII

10 *µ*l measurement sample was mixed with 40 *µ*l Owren Veronal buffer solution, and 50 *µ*l of factor VIII-deficient plasma (Sysmex Corporation) was added thereto and heated at 37 *µ*C for 1 minute. Then, 50 *µ*l Thrombocheck APTT-SLA (Sysmex Corporation) was added to the mixture after heating and then kept at 37°C for 3 minutes, and after 50 *µ*l of 20 mM CaCl2 was added thereto, the coagulation time was measured by a blood coagulation analyzer Coaglex 800 (Shimadzu Corporation).

### (3) Measurement of factor VIII

Measurement of factor VIII was carried out according to a method described in British Journal of Haematology, 86:106-111, 1994. In the method described therein, factor VIII in a measurement sample is detected by ELISA using a polyclonal antibody against factor VIII.

### (4) Measurement of VWF

10 *µ*l measurement sample was heated at 37°C for 3 minutes, and then 200 *µ*l VWF reagent buffer solution (R1) (Dade-Behring) was added thereto and heated at 37°C for 5 minutes. Then, 100 *µ*l VWF reagent latex solution (R2) (Dade-Behring) was added thereto, and the resulting mixture was measured for absorbance at 700 nm by Coaglex 800 (Shimadzu Corporation).

### (5) Measurement of the activity of VWF Rco

100 µl measurement sample was collected on a slide judgment plate, and 200 *µ*l VWF Ristocetin Cofactor Activity Measurement Reagent (Dade-Behring) was dropped thereon. The slide judgment plate was jiggled for 3 minutes, and the degree of coagulation was judged with naked eyes. As a blank, physiological saline was used, and as a standard sample, normal human plasma SHP (Dade-Behring) was used. The degree of coagulation was judged as "4+" when the coagulation was the same as in the normal human plasma SHP stock solution, "3+" when the coagulation was the same as in a 2-fold dilution of the normal human plasma SHP, "2+" when the coagulation was the same as in a 4-fold dilution of the normal human plasma SHP, "1+" when the coagulation was the same as in a 8-fold dilution of the normal human plasma SHP, and "-" when the coagulation was the same as, or lower than, in a 16-fold dilution of the normal human plasma SHP.

### Example 1

The activity of factor VIII obtained by contact treatment of human plasma with cation exchange resin was measured. Eight kinds of cation exchange resin shown in Table 1 were used in this example.

**Table 1**

| Type of cation exchange resin | | Manufacturer |
|---|---|---|
| Name | Type of functional group | |
| Amberlite IR120B Na | sulfonate type | Organo Corporation |
| Amberlite IR124 Na | sulfonate type | Organo Corporation |
| Amberlite 200CT Na | sulfonate type | Organo Corporation |
| Amberlite IRC50 | carboxylate type | Organo Corporation |
| Amberlite IRC76 | carboxylate type | Organo Corporation |
| Amberlite IRC748 | iminodiacetate type | Organo Corporation |
| Muromac A-1 | iminodiacetate type | Muromachi Chemicals INC |
| MuromacB-1 | iminodiacetate type | Muromachi Chemicals INC |

The contact treatment was carried out by mixing 10 ml human plasma with 1 g (10% (w/v)) of each cation exchange resin shown Table 1 and then stirring the mixture at room temperature for 2 hours. The activity of factor V and the activity of factor VIII in the plasma obtained by the contact treatment were measured according to the measurement methods (1) and (2) described above.

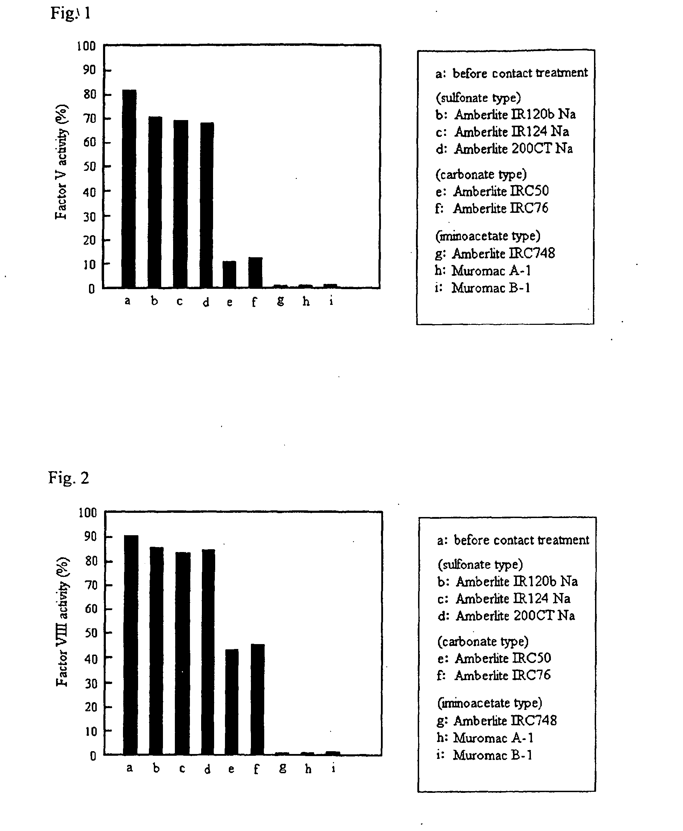

The measurement results of the activity of factor VIII are shown in Fig. 1, and the measurement results of the activity of factor V are shown in Fig. 2. The activity (%) of factor VIII or the activity (%) of factor (V) in each measurement sample was calculated assuming that the coagulation activity obtained by measuring a standard sample not subjected to the contact treatment to be 100%. As the standard preparation, Coagtrol N (Sysmex Corporation) was used. Figs. 1 and 2 show the activity (%) of factor VIII and the activity (%) of factor V respectively on the ordinate. In each graph, a indicates a result obtained by using, as a measurement sample, human plasma not subjected to contact treatment; b, plasma obtained by subjecting human plasma to contact treatment with Amberlite IR120B Na; c, plasma obtained by subjecting human plasma to contact treatment with Amberlite IR124 Na; d, plasma obtained by subjecting human plasma to contact treatment with Amberlite 200CT Na; e, plasma obtained by subjecting human plasma to contact treatment with Amberlite IRC50; f, plasma obtained by subjecting human plasma to contact treatment with Amberlite IRC76; g, plasma obtained by subjecting human plasma to contact treatment with Amberlite IRC748; h, plasma obtained by subjecting human plasma to contact treatment with Muromac A-1; i, plasma obtained by subjecting human plasma to contact treatment with Muromac B-1.

Figs. 1 and 2 show that when cation exchange resin having a sulfonate group was used in the contact treatment (b to d), both factors V and VIII maintained a majority of their activity. When cation exchange resin having a carboxylate group was used in the contact treatment (e, f), the activity of factor V could be reduced to about 10%, while about 40% of the activity of factor VIII remained. On the other hand, when cation exchange resin having an iminodiacetate group was used in the contact treatment (g to i), both factors V and VIII lost most of their activity.

In addition to the activity of factor VIII, the contents of factor VIII and VWF and the activity of VWF Rco in each plasma obtained by contact treatment with the cation exchange resin having an iminodiacetate group were examined according to the measurement methods (3), (4) and (5) described above.

The measurement results are shown in Table 2. The activity (%) of factor VIII in each measurement sample was calculated assuming that the coagulation activity obtained by measuring human plasma not subjected to the contact treatment to be 100%. The contents (%) of factor VIII and VWF in each measurement sample were calculated assuming that the measurement results obtained by measuring human plasma not subjected to the contact treatment to be 100%, respectively.

**Table 2**

| Cation exchange resin | Factor VIII activity (%) | Factor VIII content (%) | VWF content (%) | VWFRco activity |
|---|---|---|---|---|
| Muromac A-1 | 0.58 | 95.3 | 76.8 | 4+ |
| Muromac B-1 | 0.29 | 86.1 | 70.0 | 4+ |
| Amberlite IRC748 | 0.29 | 57.1 | 42.5 | 3+ |

From Table 2, it was found that in any plasma obtained by the contact treatment, factor VIII remained although a majority of the activity of factor VIII was lost. It was further found that VWF also remained, and the VWF had an Rco activity.

### Example 2

The activities of factors V and VIII in plasma obtained by contact treatment with cation exchange resin having an iminodiacetate group were measured.

The contact treatment was carried out by mixing 100 ml human plasma with cation exchange resin having an iminodiacetate group (Muromac A-1) at a cation exchange resin concentration of 0, 2.5, 5.0, 7.5, and 10.0% (w/v), respectively. The activities of factors VIII and V before the contact treatment and in each treatment time (in treatment times of 0.5, 1.0, 1.5 and 2.0 hours respectively) were measured according to the measurement methods (1) and (2) described above.

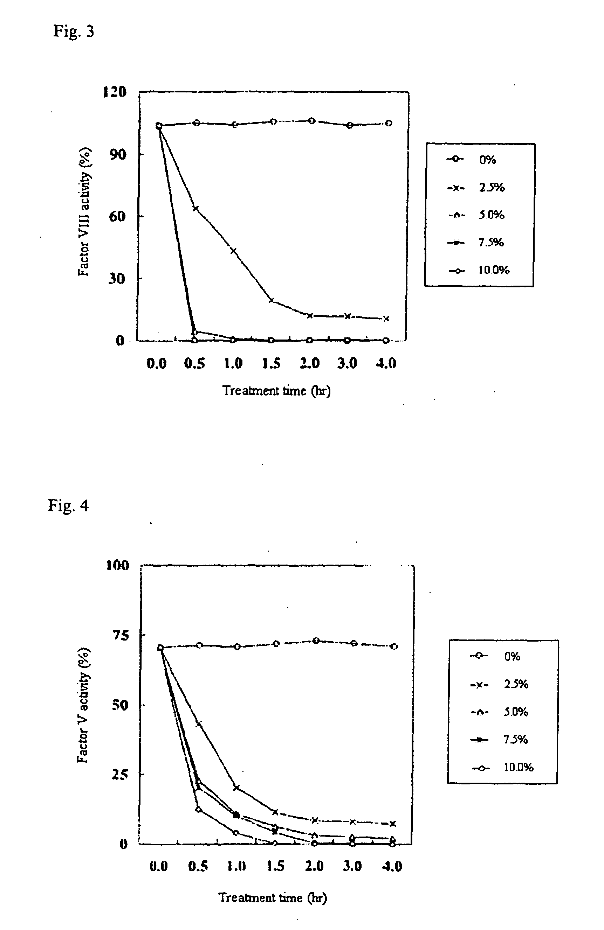

The measurement results of the activity of factor VIII are shown in Fig. 3, and the measurement results of the activity of factor V are shown in Fig. 4. The activity (%) of factor VIII or the activity (%) of factor (V) in each measurement sample was calculated assuming that the coagulation activity obtained by measuring a standard sample not subjected to the contact treatment to be 100%. As the standard preparation, Coagtrol N (Sysmex Corporation) was used. Figs. 3 and 4 show the activity (%) of factor VIII and the activity (%) of factor V respectively on the ordinate. Each graph shows treatment times on the abscissa.

From Figs. 3 and 4, it was found that the activities of both factors V and VIII were reduced by the contact treatment with cation exchange resin having an iminodiacetate group. It was found that the activities of both factors V and VIII were reduced to 1% or less by the treatment for 30 minutes or more, preferably 2 hours or more, under the conditions where the concentration of the cation exchange resin was 5% (w/v) or more.

### Example 3

It was examined whether factor VIII in plasma congenitally deficient in only factor V, and factor V in plasma congenitally deficient in only factor VIII, were inactivated respectively by contact treatment with cation exchange resin having an iminodiacetate group.

Factor V-deficient plasma (4 cases: patients 1 to 4) and factor VIII-deficient plasma (4 cases: patients 5 to 8) were purchased from George King Bio-Medical, INC. The contact treatment was carried out by mixing cation exchange resin having an iminodiacetate group (Muromac A-1) in an amount of 10.0% (w/v) with 1 ml of each plasma. The activity of factor VIII or V in each plasma before the contact treatment and in each treatment time (in treatment times of 1.0, 2.0, 3.0 and 4.0 hours respectively) was measured according to the measurement methods (1) and (2) described above.

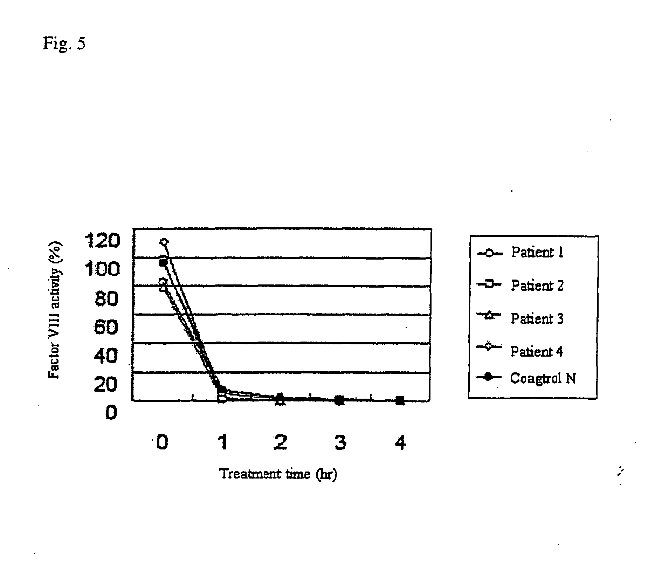

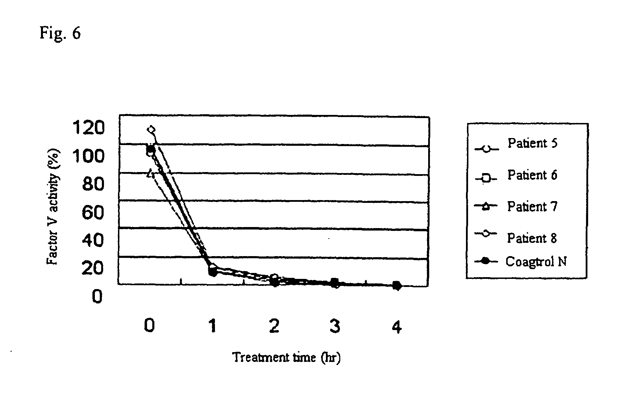

The measurement results of the activity of factor VIII in the factor V-deficient plasma are shown in Fig. 5, and the measurement results of the activity of factor V in the factor VIII-deficient plasma are shown in Fig. 6. The activity (%) of factor VIII or the activity (%) of factor V in each measurement sample was calculated assuming that the coagulation activity obtained by measuring a standard sample not subjected to the contact treatment to be 100%. As the standard preparation, Coagtrol N (Sysmex Corporation) was used. Figs. 5 and 6 show the activity (%) of factor VIII and the activity (%) of factor V respectively on the ordinate. Each graph shows treatment times on the abscissa.

In Fig. 5, the activity of factor VIII in the factor V-deficient plasma was reduced to about 10% in 1 hour of the contact treatment, and then to less than 1% in 2 to 4 hours of the contact treatment. In Fig. 6, the activity of factor V in the factor VIII-deficient plasma was reduced to about 10% in 1 hour of the contact treatment, and then to less than 1 % in all plasma samples in 4 hours of the contact treatment.

From the foregoing, it was found that the factors V and VIII were changed independently into an inactivated form by the contact treatment with cation exchange resin having an iminodiacetate group.

### Example 4

Confact F (100 U/ml, KAKETSUKEN, Japan), that is, a factor VIII/VWF complex, was dissolved in physiological saline to give a factor VIII complex solution (10000%; 1 U/ml corresponds to 100%) which was then subjected to contact treatment for 4 hours with 20% (w/v) cation exchange resin having an iminodiacetate group. According to the measurement methods (2), (3), (4) and (5) described above, the solution obtained by this contact treatment was measured for factor VIII activity, factor VIII, VWF, and VWF Rco activity. Separately, normal human plasma was subjected to the same contact treatment, and the resulting plasma was measured for factor VIII activity, factor VIII, VWF, and VWF Rco activity.

The results are collectively shown in Table 3. The activity (%) of factor VIII in the factor VIII complex solution subjected to the contact treatment was calculated assuming that the coagulation activity obtained by measuring the factor VIII complex solution not subjected to the contact treatment was 100%, and when the activity to be 1% or more, "+" was given, and when the activity was less than 1%, "-" was given. The activity (%) of factor VIII in the normal human plasma subjected to the contact treatment was calculated assuming that the coagulation activity obtained by measuring the normal human plasma not subjected to the contact treatment to be 100%, and when the activity was 1% or more, "+" was given, and when the activity was less than 1%, "-" was given. The content (%) of factor VIII and the content (%) of VWF in the factor VIII complex solution subjected to the contact treatment were calculated assuming that the measurement results obtained by measuring the factor VIII complex solution not subjected to the contact treatment were 100%, and when the content to be 70% or more, "+" was given, and when the content was less than 70%, "-" was given. The content (%) of factor VIII and the content (%) of VWF in the normal human plasma subjected to the contact treatment were calculated assuming that the measurement results obtained by measuring the normal human plasma not subjected to the contact treatment were 100%, and when the content to be 70% or more, "+" was given, and when the content was less than 70%, "-" was given.

**Table 3**

| | Factor VIII activity | Factor VIII | VWF | VWFRco activity |
|---|---|---|---|---|
| Factor VIII complex solution | - | + | + | 4+ |
| Normal human plasma | - | + | + | 4+ |

From Table 3, it was found that in the factor VIII complex solution and normal human plasma obtained by the contact treatment, the activity of factor VIII was lost, but factor VIII remained. It was further found that VWF also remained, and the VWF had an Rco activity. That is, it could be confirmed that the influence of the contact treatment on the factor VIII/VWF complex shows the same behavior as in the influence on the normal human plasma.

### Example 5

A solution of Kogenate FS (250 U/ml, Bayer Yakuhin, Ltd.), that is, pure factor VIII, in physiological saline (25000%), and a solution obtained by contact treatment of the solution of Kogenate FS for 4 hours with 20% (w/v) cation exchange resin having an iminodiacetate group, were subjected to SDS electrophoresis under reducing and non-reducing conditions.

Further, a solution of human factor V (50 *µ*g/ml, Hematologic Technology Inc.) in physiological saline (15000%), and a solution obtained by contact treating the solution of human factor V for 4 hours with 20% (w/v) cation exchange resin having an iminodiacetate group, were subjected to SDS electrophoresis under reducing and non-reducing conditions.

SDS polyacrylamide gel electrophoresis (hereinafter, SDS-PAGE) was conducted with MINI-PROTEIN II electrophoresis unit (Nippon Bio-Rad Laboratories). Under the reducing conditions, SDS-PAGE was conducted as follows: The solution was diluted 4-fold with 1% (w/v) SDS solution, and the resulting dilution was treated with 5% (w/v) 2-mercaptoethanol at 56°C for 30 minutes, and then developed at 60V for 2.5 hours on 7.5% polyacrylamide gel containing 0.1% (w/v) SDS. Under the non-reducing conditions, on the other hand, the sample was similarly diluted 4-fold with 1% (w/v) SDS solution, and the resulting dilution was developed at 60V for 2.5 hours on 7.5% polyacrylamide gel containing 0.1% (w/v) SDS. As the buffer solution, 25 mM Tris-192 mM glycine buffer (pH 7.5) containing 0.02% (w/v) SDS was used. For staining, 2D silver staining reagent II (Daiichi Pure Chemicals Co., Ltd.) was used.

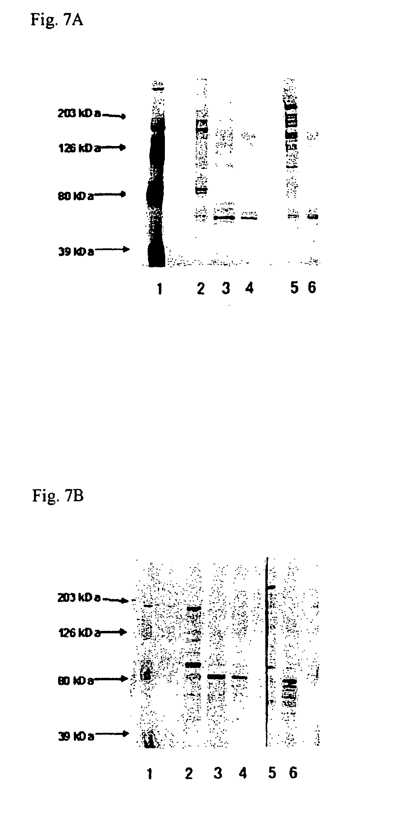

The resulting electrophoresis photograph is shown in Fig. 7. Fig. 7A is an electrophoresis photograph obtained by electrophoresis under the non-reducing conditions, and Fig. 7B is an electrophoresis photograph obtained by electrophoresis under the reducing conditions. In each photograph, lane 1 is a molecular-weight marker, lane 2 is a solution containing factor VIII (Kogenate FS) before the contact treatment, lanes 3 and 4 are solutions obtained by contact treatment of a solution containing factor VIII (Kogenate FS), lane 5 is a solution containing human factor V before the contact treatment, and lane 6 is a solution obtained by contact treatment of a solution containing human factor V

In lanes 3, 4 and 6 in Figs. 7A and 7B, protein bands could be confirmed. Thereby, it could be confirmed that factors V and VIII remained in the solutions even after the contact treatment.

### Example 6

In this example, plasma obtained by collecting blood from healthy beagles was used. This normal canine plasma was subjected to contact treatment for 4.5 hours with 20% (w/v) cation exchange resin having an iminodiacetate group (Muromac A-1). The activities of factors VIII and V in the plasma obtained by this contact treatment were measured according to the measurement methods (1) and (2) described above.

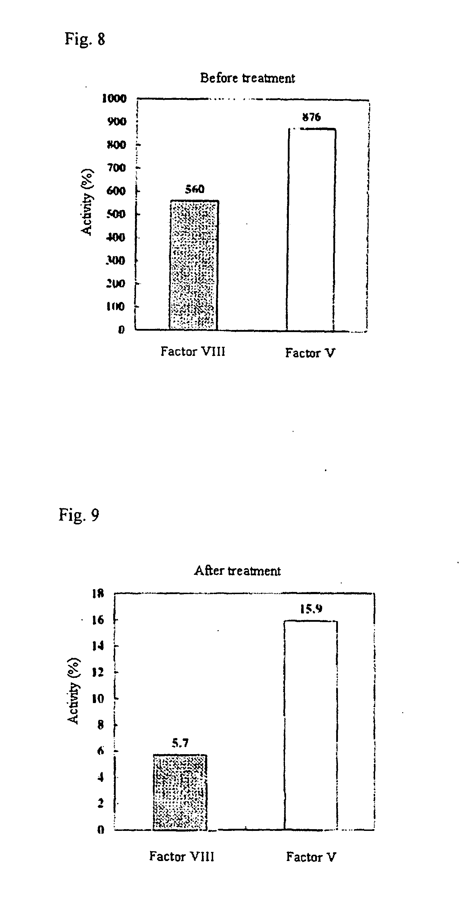

The measurement results of the activities of factors VIII and V in the normal canine plasma before the contact treatment are shown in Fig. 8, and the measurement results of the activities of factors VIII and V in the plasma obtained by contact treatment of the normal canine plasma are shown in Fig. 9. The activity (%) of factor VIII or the activity (%) of factor V in each measurement sample was calculated assuming that the coagulation activity obtained by measuring a standard sample not subjected to the contact treatment to be 100%. As the standard preparation, Coagtrol N (Sysmex Corporation) was used. Each graph shows the activity of each blood coagulation factor on the ordinate.

In Fig. 8, the activities of factors VIII and V in the normal canine plasma showed 560% and 876%, respectively. These activities were higher about 6- and 9-times as compared with those in humans. In Fig. 9, however, the activities of factors VIII and V in the plasma were reduced to 5.7% and 15.9% respectively by the contact treatment. From the foregoing, it was found that the factors VIII and V not only in human plasma but also in canine plasma are changed into an inactivated form by the contact treatment.

### Example 7

The activities of factors V and VIII in plasma obtained by contact treatment with Sepharose having an iminodiacetate group were measured. The contact treatment was carried out by mixing 100 ml normal human plasma with Sepharose having an iminodiacetate group, that is, Chelating Sepharose Fast Flow (Amersham Biosciences), at a Sepharose concentration of 0, 5.0, 10.0, and 20.0% (w/v) respectively. The activities of factors VIII and V before the contact treatment and in each treatment time (in treatment times of 1.0, 2.0 and 4.0 hours respectively) were measured according to the measurement methods (1) and (2) described above.

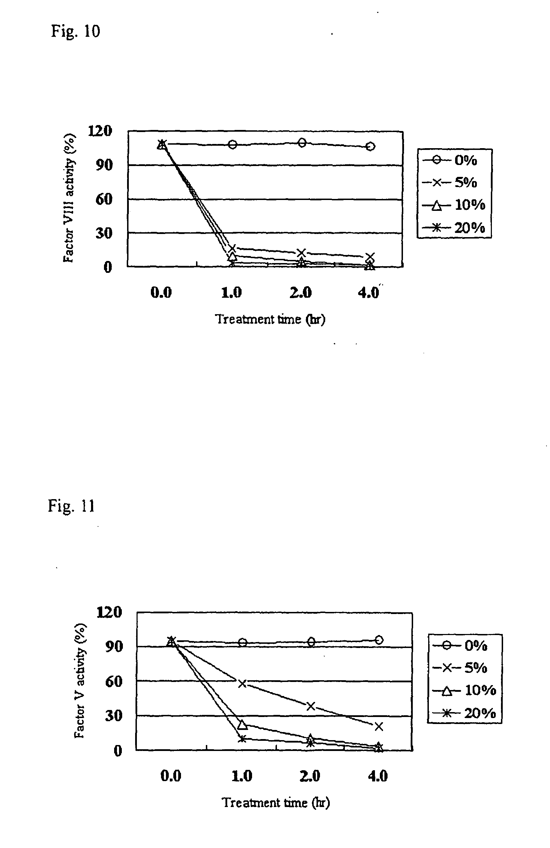

The measurement results of the activity of factor VIII are shown in Fig. 10, and the measurement results of the activity of factor V are shown in Fig. 11. The activity (%) of factor VIII or the activity (%) of factor V in each measurement sample was calculated assuming that the coagulation activity obtained by measuring a standard sample not subjected to the contact treatment to be 100%. As the standard preparation, Coagtrol N (Sysmex Corporation) was used. Figs. 10 and 11 show the activity (%) of factor VIII and the activity (%) of factor V respectively on the ordinate. Each graph shows treatment times on the abscissa.

In Fig. 10, the activity of factor VIII was reduced to about 10% in 1 hour of the contact treatment, and then to about 1 to 5% in 2 to 4 hours of the contact treatment. In Fig. 11, the activity of factor V was reduced to about 20 to 60% in 1 hour of the contact treatment, and then to 20% at a Sepharose concentration of 5.0% (w/v) or to less than 1% at a Sepharose concentration of 10.0% (w/v) or 20.0 (w/v) in 4 hours of the contact treatment. From the foregoing, it was found that the activities of factors V and VIII in the plasma were reduced by the contact treatment with Sepharose having an iminodiacetate group.

Prior to use, Sepharose having an iminodiacetate group was pre-treated with a solution of sodium hydroxide in this example. By this pre-treatment, R in the iminodiacetate group (-N(CH₂COOR)₂) possessed by Sepharose is changed from hydrogen atom to sodium ion. When measurement results obtained by the contact treatment with pre-treated Sepharose having an iminodiacetate group (R in the iminodiacetate group is sodium ion) are compared with measurement results obtained by the contact treatment with Sepharose having an iminodiacetate group (R in the iminodiacetate group is hydrogen atom) not subjected to pre-treatment, the activities of both factors V and VIII are reduced to low levels in a shorter time with Sepharose subjected to pre-treatment (not shown in the figures). From the foregoing, it can be seen that the factors V and VIII can be changed into an inactivated form more effectively by contact treatment with Sepharose having an iminodiacetate group (-N(CH₂COOR)₂) wherein R is sodium ion than with Sepharose wherein R is hydrogen atom.

### Example 8

Confact F (100 U/ml, KAKETSUKEN, Japan), that is, a factor VIII/VWF complex, was dissolved in physiological saline to give a factor VIII complex solution (10000%; 1 U/ml corresponds to 100%) which was then subjected to contact treatment for 4 hours with 20% (w/v) Sepharose having an iminodiacetate group. According to the measurement methods (2), (3), (4) and (5) described above, the solution obtained by this contact treatment was measured for factor VIII activity, factor VIII, VWF, and VWF Rco activity. Separately, normal human plasma was subjected to the same contact treatment, and the resulting plasma was measured for factor VIII activity, factor VIII, VWF, and VWF Rco activity.

The results are shown in Table 4. The activity (%) of factor VIII in the factor VIII complex solution subjected to the contact treatment was calculated assuming that the coagulation activity obtained by measuring the factor VIII complex solution not subjected to the contact treatment to be 100%, and when the activity was 1% or more, "+" was given, and when the activity was less than 1%, "-" was given. The activity (%) of factor VIII in the normal human plasma subjected to the contact treatment was calculated assuming that the coagulation activity obtained by measuring the normal human plasma not subjected to the contact treatment to be 100%, and when the activity was 1% or more, "+" was given, and when the activity was less than 1%, "_" was given. The content (%) of factor VIII and the content (%) of VWF in the factor VIII complex solution subjected to the contact treatment were calculated assuming that the measurement results obtained by measuring the factor VIII complex solution not subjected to the contact treatment to be 100%, and when the content was 70% or more, "+" was given, and when the content was less than 70%, "-" was given. The content (%) of factor VIII and the content (%) of VWF in the normal human plasma subjected to the contact treatment were calculated assuming that the measurement results obtained by measuring the normal human plasma not subjected to the contact treatment were 100%, and when the content to be 70% or more, "+" was given, and when the content was less than 70%, "-" was given.

**Table 4**

| | Factor VIII activity | Factor VIII | VWF | VWFRco activity |
|---|---|---|---|---|
| Factor VIII complex solution | - | + | + | 4+ |
| Normal human plasma | - | + | + | 4+ |

From Table 4, it was found that in the factor VIII complex solution and normal human plasma obtained by the contact treatment, the activity of factor VIII was lost, but factor VIII remained. It was further found that VWF also remained, and the VWF had an Rco activity. That is, it could be confirmed that the influence of the contact treatment on the factor VIII/VWF complex shows the same behavior as in the influence on the normal human plasma.

## Claims

1. An in vitro method of inactivating a blood coagulation factor, comprising a step of contacting a sample containing at least one of factors V and VIII with a carrier having an iminodiacetate group -N(CH2COOR)2 wherein R represents a hydrogen atom or a metallic ion, whereby at least one of factors V and VIII in the sample is changed into an inactivated form.

2. The inactivation method according to claim 1, wherein R is a monovalent metallic ion.

3. The inactivation method according to claim 2, wherein the monovalent metallic ion is a sodium ion.

4. The inactivation method according to claim 1, wherein the carrier having the iminodiacetate group is a cation exchange resin having the iminodiacetate group or granular agarose gel having the iminodiacetate group.

5. The inactivation method according to any one of claims 1 to 4, wherein the contact treatment is carried out with the carrier in a ratio of 5% (w/v) or more to the sample.

6. The inactivation method according to any one of claims 1 to 5, wherein the sample is plasma.

7. A blood coagulation factor-inactivated sample, comprising at least one of factors V and VIII in an inactivated form obtainable by the method according to any one of claims 1 to 6.

8. The blood coagulation factor-inactivated sample according to claim 7, further comprising a von Willebrand factor.

9. The blood coagulation factor-inactivated sample according to claim 8, wherein the von Willebrand factor has a ristocetin cofactor activity.

10. A method of measuring an activity of a blood coagulation factor contained in a blood sample, comprising the steps of:
(a) preparing a mixture by mixing the blood sample, a measurement reagent for measuring a coagulation time and a blood sample treating agent comprising blood coagulation factor-inactivated plasma containing factors V and VIII in an inactivated form obtainable by the method according to any one of claims 1 to 6;
(b) measuring a coagulation time in the mixture; and
(c) calculating an activity of factor V or VIII on the basis of the coagulation time.

11. The measurement method according to claim 10, wherein the blood sample treating agent of the step (a) is the blood coagulation factor-inactivated plasma, the measurement reagent of the step (b) is a prothrombin time measurement reagent, and the step (c) calculates an activity of factor V.

12. The measurement method according to claim 10, wherein the blood sample treating agent of the step (a) is the blood coagulation factor-inactivated plasma to which factor VIII, which can be changed into an activated form is added, the measurement reagent of the step (b) is a prothrombin time measurement reagent, and the step (c) calculates an activity of factor V.

13. The measurement method according to claim 10, wherein the blood sample treating agent of the step (a) is the blood coagulation factor-inactivated plasma to which factor VIII which can be changed into an activated form is added, the measurement reagent of the step (b) is an activated partial thromboplastin time measurement reagent, and the step (c) calculates an activity of factor V.

14. The measurement method according to claim 10, wherein the blood sample treating agent of the step (a) is the blood coagulation factor-inactivated plasma to which factor V, which can be changed into an activated form is added, the measurement reagent of the step (b) is an activated partial thromboplastin time measurement reagent, and the step (c) calculates an activity of factor VIII.

15. The measurement method according to any one of claims 10 to 14, wherein the blood sample is plasma.

16. A blood sample treating agent, comprising:
(a) blood coagulation factor-inactivated plasma containing factors V and VIII in an inactivated form obtainable by the method according to any one of claims 1 to 6; and
(b) factor V which can be changed into an activated form or factor VIII which can be changed into an activated form.

## Patentansprüche

1. In-vitro-Verfahren zur Inaktivierung eines Blutgerinnungsfaktors, umfassend den Schritt des Kontaktierens einer Probe, enthaltend zumindest einen der Faktoren V und VIII, mit einem Träger, der eine Iminodiacetatgruppe -N(CH₂COOR)₂ aufweist, worin R ein Wasserstoffatom oder ein Metallion darstellt, wodurch zumindest einer der Faktoren V und VIII in der Probe in eine inaktivierte Form umgewandelt wird.

2. Inaktivierungsverfahren gemäß Anspruch 1, worin R ein monovalentes Metallion ist.

3. Inaktivierungsverfahren gemäß Anspruch 2, worin das monovalente Metallion ein Natriumion ist.

4. Inaktivierungsverfahren gemäß Anspruch 1, worin der Träger mit der Iminodiacetatgruppe ein Kationenaustauscherharz mit der Iminodiacetatgruppe oder ein granuläres Agarosegel mit der Iminodiacetatgruppe ist.

5. Inaktivierungsverfahren gemäß einem der Ansprüche 1 bis 4, worin die Kontaktbehandlung ausgeführt wird mit dem Träger in einem Verhältnis von 5 % (G/V) oder mehr in Bezug auf die Probe.

6. Inaktivierungsverfahren gemäß einem der Ansprüche 1 bis 5, worin die Probe Plasma ist.

7. Blutgerinnungsfaktor-inaktivierte Probe, umfassend zumindest einen der Faktoren V und VIII in einer inaktivierten Form, erhältlich durch das Verfahren gemäß einem der Ansprüche 1 bis 6.

8. Blutgerinnungsfaktor-inaktivierte Probe gemäß Anspruch 7, des weiteren umfassend einen von-Willebrand-Faktor.

9. Blutgerinnungsfaktor-inaktivierte Probe gemäß Anspruch 8, worin der von-Willebrand-Faktor Ristocetin-Cofaktor-Aktivität aufweist.

10. Verfahren zur Messung der Aktivität eines Blutgerinnungsfaktors, der in einer Blutprobe enthalten ist, umfassend die Schritte:
(a) Herstellen einer Mischung durch Mischen der Blutprobe, eines Meßreagens zum Messen der Gerinnungszeit und eines Blutproben-Behandlungsmittels umfassend Blutgerinnungsfaktor-inaktiviertes Plasma enthaltend die Faktoren V und VIII in einer inaktivierten Form, erhältlich durch das Verfahren gemäß einem der Ansprüche 1 bis 6;
(b) Messen der Gerinnungszeit in der Mischung; und
(c) Berechnen der Aktivität von Faktor V oder Faktor VIII auf der Basis der Gerinnungszeit.

11. Meßverfahren gemäß Anspruch 10, worin das Blutproben-Behandlungsmittel von Schritt (a) das Blutgerinnungsfaktor-inaktivierte Plasma ist, das Meßreagens von Schritt (b) ein Prothrombin-Zeitmeßreagens ist, und der Schritt (c) die Aktivität von Faktor V berechnet.

12. Meßverfahren gemäß Anspruch 10, worin das Blutproben-Behandlungsmittel von Schritt (a) das Blutgerinnungsfaktor-inaktivierte Plasma ist, zu welchem Faktor VIII, welcher in eine aktivierte Form umgewandelt werden kann, hinzugefügt wird, das Meßreagens von Schritt (b) ein Prothrombin-Zeitmeßreagens ist, und der Schritt (c) die Aktivität von Faktor V berechnet.

13. Meßverfahren gemäß Anspruch 10, worin das Blutproben-Behandlungsmittel von Schritt (a) das Blutgerinnungsfaktor-inaktivierte Plasma ist, zu welchem Faktor VIII, welcher in eine aktivierte Form umgewandelt werden kann, hinzugefügt wird, das Meßreagens von Schritt (b) ein aktiviertes partielles Thromboplastin-Zeitmeßreagens ist, und der Schritt (c) die Aktivität von Faktor V berechnet.

14. Meßverfahren gemäß Anspruch 10, worin das Blutproben-Behandlungsmittel von Schritt (a) das Blutgerinnungsfaktor-inaktivierte Plasma ist, zu welchem Faktor V, welcher in eine aktivierte Form umgewandelt werden kann, hinzugefügt wird, das Meßreagens von Schritt (b) ein aktiviertes partielles Thromboplastin-Zeitmeßreagens ist, und der Schritt (c) die Aktivität von Faktor VIII berechnet.

15. Meßverfahren gemäß einem der Ansprüche 10 bis 14, worin die Blutprobe Plasma ist.

16. Blutproben-Behandlungsmittel, umfassend:
(a) Blutgerinnungsfaktor-inaktiviertes Plasma enthaltend Faktoren V und VIII in einer inaktivierten Form, erhältlich durch das Verfahren gemäß einem der Ansprüche 1 bis 6; und
(b) Faktor V, welcher in eine aktivierte Form umgewandelt werden kann, oder Faktor VIII, welcher in eine aktivierte Form umgewandelt werden kann.

## Revendications

1. Procédé in vitro d'inactivation d'un facteur de coagulation sanguine, comprenant une étape consistant à mettre en contact un échantillon contenant au moins un des facteurs V et VIII avec un véhicule ayant un groupe iminodiacétate -N(CH2COOR)2 dans lequel R représente un atome d'hydrogène ou un ion métallique, d'où il résulte qu'au moins un des facteurs V et VIII dans l'échantillon est changé en une forme inactivée.

2. Procédé d'inactivation selon la revendication 1, dans lequel R est un ion métallique monovalent.

3. Procédé d'inactivation selon la revendication 2, dans lequel l'ion métallique monovalent est un ion sodium.

4. Procédé d'inactivation selon la revendication 1, dans lequel le véhicule ayant un groupe iminodiacétate est une résine échangeuse de cation ayant un groupe iminodiacétate ou est un gel d'agarose granulaire ayant un groupe iminodiacétate.

5. Procédé d'inactivation selon l'une quelconque des revendications 1 à 4, dans lequel le traitement de contact est réalisé avec le véhicule dans un rapport de 5 % (p/v) ou plus de l'échantillon.

6. Procédé d'inactivation selon l'une quelconque des revendications 1 à 5, dans lequel l'échantillon est le plasma.

7. Echantillon ayant un facteur de coagulation sanguine inactivé, comprenant au moins un des facteurs V et VIII dans une forme inactivée, qui peut être obtenu par le procédé selon l'une quelconque des revendications 1 à 6.

8. Echantillon ayant un facteur de coagulation sanguine inactivé selon la revendication 7, comprenant en outre un facteur de von Willebrand.

9. Echantillon ayant un facteur de coagulation sanguine inactivé selon la revendication 8, dans lequel le facteur de von Willebrand a une activité de cofacteur de la ristocétine.

10. Procédé de mesure d'une activité d'un facteur de coagulation sanguine contenu dans un échantillon sanguin, comprenant les étapes consistant à :
(a) préparer un mélange en mélangeant l'échantillon sanguin, un réactif de mesure pour mesurer un temps de coagulation et un agent de traitement de l'échantillon sanguin comprenant un plasma ayant un facteur de coagulation sanguine inactivé contenant les facteurs V ou VIII dans une forme inactivée, qui peut être obtenu par le procédé selon l'une quelconque des revendications 1 à 6 ;
(b) mesurer un temps de coagulation dans le mélange ; et
(c) calculer une activité du facteur V et VIII sur la base du temps de coagulation.

11. Procédé de mesure selon la revendication 10, dans lequel l'agent de traitement de l'échantillon sanguin de l'étape (a) est le plasma ayant un facteur de coagulation sanguine inactivé, le réactif de mesure de l'étape (b) est un réactif de mesure du temps de prothrombine, et l'étape (c) calcule une activité du facteur V.

12. Procédé de mesure selon la revendication 10, dans lequel l'agent de traitement de l'échantillon sanguin de l'étape (a) est le plasma ayant un facteur de coagulation sanguine inactivé auquel le facteur VIII, qui peut être changé en une forme activée, est ajouté, le réactif de mesure de l'étape (b) est un réactif de mesure du temps de prothrombine, et l'étape (c) calcule une activité du facteur V.

13. Procédé de mesure selon la revendication 10, dans lequel l'agent de traitement de l'échantillon sanguin de l'étape (a) est le plasma ayant un facteur de coagulation sanguine inactivé auquel le facteur VIII, qui peut être changé en une forme activée, est ajouté, le réactif de mesure de l'étape (b) est un réactif de mesure du temps de thromboplastine partielle activée, et l'étape (c) calcule une activité du facteur V.

14. Procédé de mesure selon la revendication 10, dans lequel l'agent de traitement de l'échantillon sanguin de l'étape (a) est le plasma ayant un facteur de coagulation sanguine inactivé auquel le facteur V, qui peut être changé en une forme activée, est ajouté, le réactif de mesure de l'étape (b) est un réactif de mesure du temps de thromboplastine partielle activée, et l'étape (c) calcule une activité du facteur VIII.

15. Procédé de mesure selon l'une quelconque des revendications 10 à 14, dans lequel l'échantillon de sang est un plasma.

16. Agent de traitement d'un échantillon sanguin, comprenant :
(a) un plasma ayant un facteur de coagulation sanguine inactivé, contenant les facteurs V et VIII dans une forme inactivée, qui peut être obtenue par le procédé selon l'une quelconque des revendications 1 à 6 ; et
(b) le facteur V qui peut être changé en une forme activée ou le facteur VIII qui peut être changé en une forme activée.
